# EUROPEAN PATENT SPECIFICATION

(11) **EP 0 934 731 B1**
(45) Date of publication and mention of the grant of the patent: **03.11.2004**
(21) Application number: 98309364.2
(22) Date of filing: 16.11.1998
(51) Int. Cl.: A61B 17/80

(54) **Bone fracture fixation devices**
Fixationsvorrichtungen für Knochenfrakturen
Dispositifs pour la fixation des fractures des os

(30) Priority: 18.11.1997 GB 9724322
(43) Date of publication of application: 11.08.1999
(73) Proprietor: The University of Hull, Kingston-upon-Hull HU6 7RX (GB)
(72) Inventor: Fagan, Michael John, East Yorkshire YO25 8SH (GB); Bowen, Gareth, Abergavenny Gwent NP7 5LR (GB); Mohsen, Amr Mohamed Mohamed Abdel, Swanland East Yorkshire HU14 3RG (GB)
(74) Representative: Hartley, Andrew Philip

(56) References cited:
- GB-A- 1 517 161
- GB-A- 2 158 716
- US-A- 4 364 382
- US-A- 5 015 248

## Description

The invention relates to fracture fixation devices.

One type of fracture fixation device is suitable for fixing femurs that have fractured subsequently to the fitting to the femur of a prosthesis which forms part of an artificial hip or knee joint. "Fixing", as used herein, means holding the femur (or other bone) in a suitable configuration for healing of the fracture.

The fixation of a femur that has fractured subsequently to the fitting of a prosthesis to the femur presents particular difficulties. In particular, it is undesirable to insert screws into the femur near the prosthesis as this can further damage the cement holding the prosthesis to the femur and cause loosening of the prosthesis. For this reason many common fixation plates, which are secured to the bone solely by screws, are unsuitable. Furthermore, since the screws cannot pass through the metal prosthesis they would therefore have to pass either side of it, reducing the purchase on the screw.

A fixation device that has been used to fix femurs fitted with prostheses is described in an article by E J Zenni et al. published in "Clinical Orthopaedics and Related Research", number 231 (June 1988), pp 83-89. This device comprises an elongate body provided with a plurality of holes at one end and a plurality of grooves at the other end. The end of the body provided with the grooves is locked to the femur, proximally to the prosthesis, by bands which pass around the shaft of the femur and the body and locate in the grooves. The end of the body provided with the holes is locked to the femur distally from the prosthesis by screws passing through the holes. The body bridges the fracture and holds the femur in a desired configuration. As the screws are spaced from the prosthesis, damage to the cement is less likely.

However, the use of bands passing around the shaft of the femur acts as a tourniquet restricting the periosteal circulation, leading to cortical erosion.

A further fracture fixation device is disclosed in US-A-4,364,382. This device comprises an elongate body with first and second rows of fingers. Each row extends along the body and each finger of each row projects from the body. In use, the body is positioned adjacent and along an elongate bone, bridging a fracture, with the fingers of the two rows bent around the bone in respective opposite senses to hold the body to the bone.

However, this device is not suitable for fixing femoral fractures because it provides insufficient flexural and torsional support to the femur. Furthermore, the device has to be sufficiently ductile to allow the fingers to be deformed around and into the bone during crimping. Therefore this device cannot be spring loaded, to grip the bone, thus the fingers can more easily become loose.

According to an aspect of the invention, there is provided a fracture fixation device for an elongate bone comprising an elongate body, first and second rows of fingers, each row extending along the body and each finger of each row projecting from the body, the body being positionable adjacent and along an elongate bone with the fingers of the two rows extending around the bone in respective opposite senses to hold the body to the bone, and two projections projecting from the body at or near a first end of the body, the projections extending around the bone in respective opposite senses, each projection having a hole for receiving a respective screw inserted into the bone to lock the projection to the bone.

The provision of the two projections allows the device to provide greater torsional and flexural support to a bone and, when dynamic compression screws are used, facilitates compression of the fracture site. When projections are provided at one end only of the body, the device may be used to fix femurs fractured in close proximity to a femoral prosthesis. In this case, the end provided with the projections is secured distally to the prosthesis.

Preferably, the two projections are of different lengths.

The following is a more detailed description of embodiments of the invention, by way of exampe, reference being made to the appended schematic drawings in which:-
Figure 1 is a perspective representation of a first fracture fixation device secured to a femur;
Figure 2 is a side elevation of the device of Figure 1;
Figure 3 is a front elevation of the fixation device shown in Figures 1 and 2;
Figure 4 is a front elevation of the device of Figures 1 to 3 during a first stage of manufacture of the device;
Figure 5 is an end view corresponding to Figure 4;
Figure 6 is a side elevation corresponding to Figure 4;
Figure 7 is a front elevation of the device of Figures 1 to 3 during a second stage of manufacture of the device;
Figure 8 is an end elevation corresponding to Figure 7;
Figure 9 is a side elevation of a spike of the device of Figures 1 to 3;
Figure 10 is an end view of a second fracture fixation device showing a bone in cross-section and showing the fingers of one row in a first configuration (solid line) corresponding to when the device is fitted to a bone and in a second configuration (dashed line) corresponding to when the fingers are in their resting position, before the device is fitted to a bone;
Figure 11 is a perspective view of a third fracture fixation device;
Figure 12 is a perspective view of a fourth fracture fixation device;
Figure 13 is a front elevation of the device of Figure 12;
Figure 14 is a side elevation of the device of Figures 12 and 13;
Figure 15 is a sectional view taken on the line A-A in Figure 14;
Figure 16 is a perspective view of a fifth fracture fixation device;
Figure 17 is a side elevation of the fifth device;
Figure 18 is a plan view of the fifth device;
Figure 19 is a perspective view of the fifth device attached to the femur in a different way; and
Figure 20 is a perspective view of a sixth fracture fixation device.

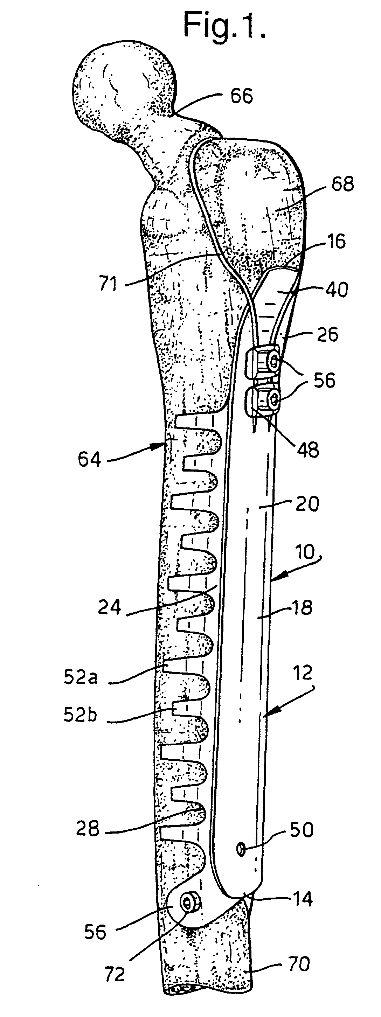

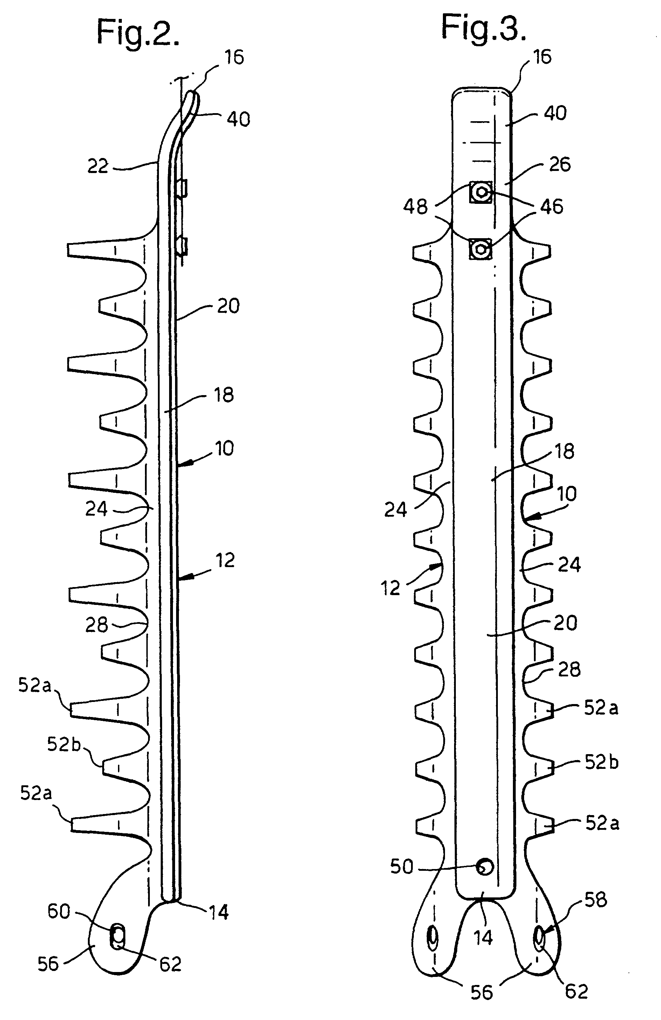

As seen in Figures 1 to 3, the first fixation device 10 has an elongate body 12 having a first end 14 and a second end 16. The body 12 has a central portion 18 which extends the full length of the body 12 and which has first and second opposed surfaces 20, 22. The body 12 also has two lateral portions 24 located at opposite sides of the central portion 18. The lateral portions 24 extend from the first end 14 of the body along the majority of the length of the body 14 stopping short of the second end 16. Hence, a region 26 of the body 12 lying at the second end 16 is formed solely from the central portion 18.

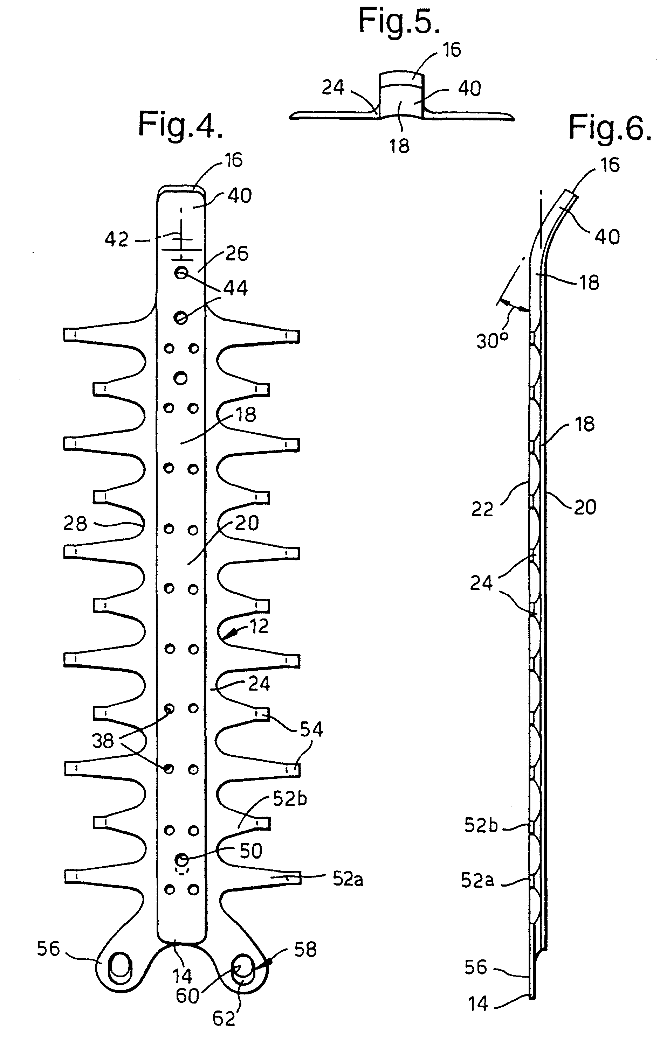

As shown most clearly in Figures 4 to 6 (which show the fixation device at a first stage of manufacture), the central portion 18 is thicker than the lateral portions 24 which taper as they extend from the central portion 18 to respective side edges 28 of the body 12.

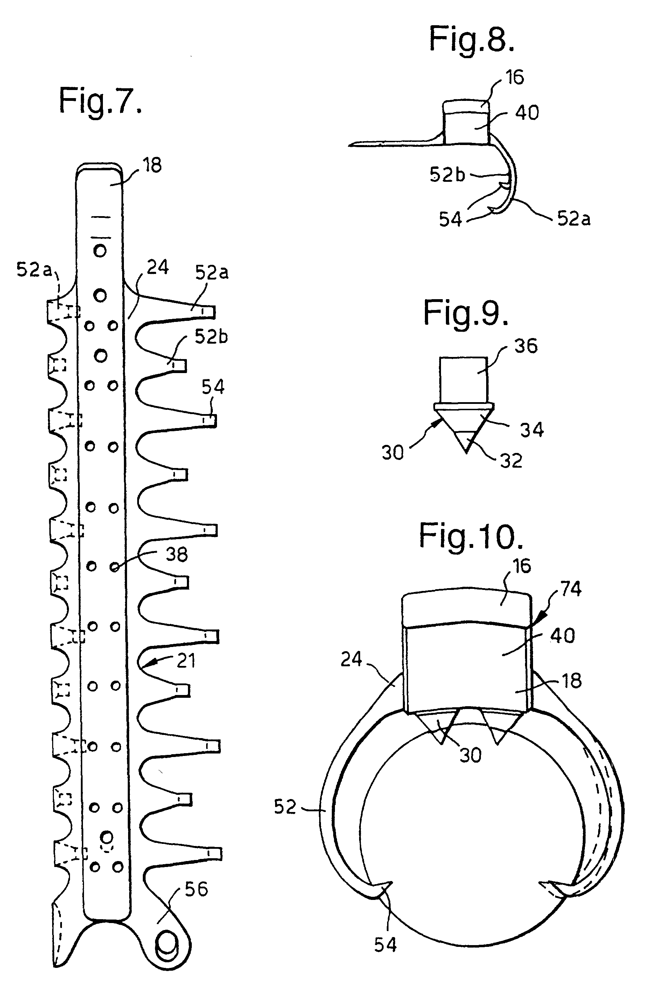

A plurality of spikes (not shown in Figures 1 to 6) extend from the second surface 22 of the central portion 18. One of the spikes 30 (which are formed separately from the body 12) is shown in isolation in Figure 9. The point of each spike 30 is formed by a conical portion 32 which extends from the point to the smaller diameter end of a frustro-conical portion 34 that is co-axial with the conical portion 32. The cone angle of the frustro-conical portion 34 is greater than the cone angle of the conical portion 32. This arrangement serves a purpose described below.

Figure 9 also shows a shank 36 of the spike 30. During manufacture, the shanks 36 of the spikes 30 are fixed into respective holes 38 provided in the central portion 18 and shown in Figure 4. The spikes 30 project from the second surface 22 is a manner similar to that shown in Figure 10 which shows another fixation device. As indicated in Figure 4, the spikes 30 are arranged in two rows which extend from the first end 14 of the body 12 for the majority of the length of the body 12 stopping short of the end region 26.

The end region 26 is bent so that a portion 40 of the body 12 including the second end 16 lies at an angle of about 30° to the longitudinal axis 42 of the body 12. The angled portion 40 is located at the opposite side of the axis 42 to the spikes 30.

The body 12 is provided with first and second threaded holes 44 near the second end 16. Each threaded hole 44 receives a respective screw 46 carrying a respective clamping part 48. The arrangement is such that a wire can be clamped between each part 48 and the body 12.

A recessed hole 50 extends through the body 12 near the first end 14 and serves a purpose described below.

From each side edge 28 project a respective plurality of fingers 52a, 52b. The fingers 52a, 52b of each side edge 28 are arranged in a row extending along the length of the body 12. The fingers 52a, 52b of each row are of longer and shorter lengths - the longer fingers 52a and the shorter fingers 52b alternating with each other along the length of the row. As best seen at the right-hand side of Figure 8 (which shows a second stage of manufacture of the fixation device), the fingers 52a, 52b are generally arcuate in shape and extend to the same side of the longitudinal axis 42 as the spikes 30. The fingers 52a, 52b lie in planes normal to the longitudinal axis 42. The arrangement is such that the fingers 52a, 52b define a channel that lies between the fingers 52a, 52b that extend from one of the side edges 28 and the fingers 52a, 52b that extend from the other one of the side edges 28. Each finger 52a, 52b has a sharp formation 54 at the end of the finger remote from the body 12. As best shown at the right-hand side of Figure 8, the sharp formations 54 project into the channel. The fingers 52a, 52b are thinner and more flexible than the central portion 18 and are resilient.

Two projections 56 extend from respective ones of the side edges 28 at the first end 14 of the body 12. Each projection 56 extends beyond the first end 14 in a direction parallel to the longitudinal axis 42 and also arcuately to the same side of the axis 42 as the spikes 30. Each projection 56 lies to a respective side of the channel formed by the fingers 52a, 52b. Each projection 56 is of plate-like construction with spaced curved side edges leading away from the body 12 and terminating in a rounded end.

Each projection 56 is provided with a hole 58 of a type known as a dynamic compression hole. Each hole 58 is bounded by an annular surface 60 that faces into the hole 58 and which has a portion 62, located furthermost from the body 12 in a direction parallel to the axis 42, that extends at an angle between opposed faces of the corresponding projection 56. These angled portions 62 of the holes 58 serve a purpose described below.

Figures 4 to 6 show a first stage of the manufacture of the fixation device 10 and Figures 7 and 8 show a second stage of its manufacture. As indicated in Figures 4 to 6, the body 12, the fingers 52a, 52b and the projections 56 are machined from a single sheet of surgical stainless steel or other suitable surgical material. The angled portion 40 is then bent away from the longitudinal axis 42 and the fingers 52a, 52b are bent into arcuate shapes to form the channel as shown at the right-hand side of Figure 8. The sharp formations 54 are then bent inwardly. The projections 56 are also bent into an arcuate shape as shown (for one side only) in Figure 7.

The shanks 36 of the spikes 30 are then fitted into the holes 38 and the screws 46 and the clamping part 48 are fitted to the first and second threaded holes 44.

The fracture fixation device is used as follows. Figure 1 shows a femur 64 to which a femoral prosthesis 66 (which forms part of a hip joint) has been fitted. The prosthesis 66 has been inserted into the medullary canal. The prosthesis 66 is held in place by cement (not shown) or another suitable material.

The first fixation device 10 described above is particularly useful for fixing such a femur when the femur is fractured proximally (close to the prosthesis 66). Firstly, the fracture site is exposed, cleared of debris and the fracture is then reduced and possibly held in place by K-wires or clamps or other fixation devices. The fracture fixation device 10 is then positioned with the body 12 adjacent to and extending along the shaft 70 of the femur 64 so that the body 12 bridges the fracture and the angled portion 40 lies against the trochanter 68. In order to do this, it is necessary to force the fingers 52a, 52b extending from one of the side edges 28 away from the fingers 52a, 52b extending from the other of the side edges so that the femoral shaft 70 can be inserted into the channel between the fingers. The fingers 52a, 52b are then released so that the fingers 52a, 52b extend around the shaft 70 in respective opposite directions, the points of the sharp formations 54 lying against the shaft 70 but not penetrating the shaft 70. At this stage, the points of the spikes 30 lie against but do not penetrate the shaft 70.

The second end 16 of the body 12 is then locked to the trochanter 68 by passing a K-wire 71 around the head of the trochanter and clamping each end of the wire 71 between each one of the clamping parts 48 and the body 12. This prevents the fixation device 10 from moving along the femoral shaft 70 away from the trochanter 68. The positioning of the wire 71 around the trochanter 68 causes less damage to the periosteum and blood supply than would be caused by passing the wire 71 around the shaft 70. This is because the wire 71 passing around the trochanter 68 does not act as a tourniquet whereas passing the wire 71 around the shaft would act as a tourniquet around the shaft.

A respective screw 72 is then passed through each of the dynamic compression holes 58 and inserted three-quarters of the way into the femoral shaft 70.

The fixation device 10 preferably applies a force of a desired magnitude to the two portions of the shaft 70 lying at either side of the fracture, so as to urge the portions together. This is referred to as compressing the fracture. In order to achieve this, a tensioning device (not shown) is firstly hooked to the first end 14 of the fixation device 10 using the recessed hole 50. The tensioning device is then engaged with the femoral shaft 70 below the fixation device 10 as shown in Figure 1 and is used to apply a force between the lower portion of the femur and the device 10 that tends to push the lower portion of the femur 64 towards the portion of the femur 64 above the fracture (the upper portion being locked to the body 12 by the wire 71). During this process, the relative positions of each screw 72 and its corresponding dynamic compression hole 58 alter - the dynamic compression holes 58 being elongated in a direction parallel to the longitudinal axis 42 in order to accommodate the movement of the screw 72 within the hole 58.

When a required compressive force has been applied between the lower femur portion and the fixation device 10, the screws 72 are tightened such that the heads of the screws seat against the respective angled portions 62, thereby locking the projections 56 to the femoral shaft 70.

The arrangement is such that when either projection 56 is locked to the femur 64, the corresponding angled portion 62 lies at an acute angle to the surface of the femur 64. In this way, each angled portion 62 acts in the manner of a wedge, resisting slipping between the screw head and the angled portion 62 in a direction that would reduce the compressive force applied to the bone. This is the case independently of the position of the screws 72 within the holes 58.

As shown in Figure 1, the projections 56 now extend around the femur 64 in respective opposite senses.

Once the screws 72 have been tightened, the body 12 is knocked towards the femoral shaft 70 so that the spikes 30 penetrate the shaft. During this process, the conical portions 32 of the spikes 30 penetrate the femur 64 relatively easily. However, when the frustro-conical portions 34 contact the surface of the femur 70, the larger cone angle of the frustro-conical portion 34 resists further insertion of the spikes 30.

The fingers 52a, 52b are then crimped around the femoral shaft 70 so as to drive the sharp formations 54 into the shaft 70. This completes the fitting of the device 10 to the femur. As shown in Figure 1, the fingers 52a, 52b of the two rows are bent around the bone in respective opposite senses.

As discussed above, the fingers 52a, 52b are springy (resilient) in nature. Preferably, the fixation device is used on a bone of sufficient width so that, after fitting of the device to the bone, the fingers lie outward of their resting position so that the resilience of the fingers causes the fingers to grip the bone.

Finally the wound is closed.

The fixation device supports the femur against flexural and torsional stresses. Additionally, the device 10 applies a compressive force to the fracture aiding healing of the fracture.

The provision of the projections 56 provides two advantages. Firstly, as the projections provide two secure points of attachment of the device 10 to the femur 64 that are spaced from one another around the circumference of the femur 64, greater torsional and flexural support is provided. Secondly, the provision of the dynamic compression holes 58 in the projections 56 allows the device 10 to be used to apply a required compressive force to the fracture. As screws 72 are only used at the distal end of the body 12 (the end that is furthest from the prosthesis 66) and beyond the cement mantle, there is no risk (or only minimal risk) of damaging the cement mantle.

The spikes 30 also provide two advantages. Firstly, the penetration of the femur 64 by the spikes 30 provides additional support against torsional forces applied to the femur 64. Secondly, the spikes 30 act to space the second surface 22 of the central portion 18 away from the surface of the femur 64. This is advantageous as it limits contact between the fixation device 10 and the femur 64 and contact between a fixation device and a bone 64 can cause damage to the periosteum of the bone.

The provision of fingers 52a, 52b of different lengths, increases the support provided by the device 10 against torsional forces applied to the femur 64.

A second fracture fixation device 74 is shown in Figure 10. Parts common to the first fixation device 10 described above are given the same reference numerals and will not be described in detail.

The only difference between the second fixation device 74 and the device 10 is that all the fingers 52a, 52b of the device 74 are of the same length. Figure 10 shows the fingers 52a, 52b of the two rows bent around a femoral shaft 70 in respective opposite senses - the sharp formations 54 of the fingers 52a, 52b penetrating the shaft. Figure 10 also shows the spikes 30 penetrating the shaft 70. The dotted line in Figure 10 indicates the position of the fingers in their resting position, when the device is supplied and before being fitted to a bone. This figure shows that when, as discussed above, the device is fitted to a bone of sufficient width so that the fingers lie outward of their resting position, the resilience of the fingers causes the fingers to grip the bone.

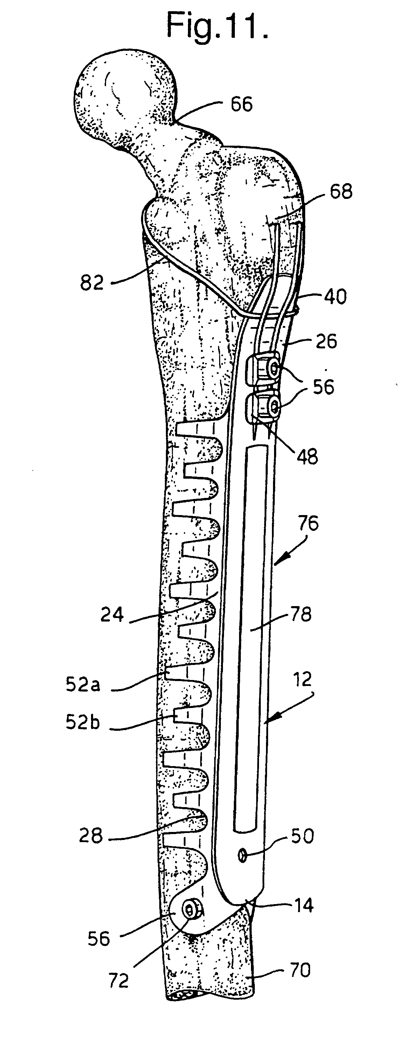

Figure 11 shows a third fracture fixation device 76. Features of the third device 76 common to the first device 10 are given the same reference numerals and are not described in detail. The third fixation device differs from the first device 10 only in that a rib 78 extends along the central portion 18. The rib 78 provides additional stiffness to the body 12. Additionally, Figure 11 shows an alternative way of attaching the first end 14 of the body 12 to the trochanter 68 using K-wires. In this case, first and second K-wires 80 each have a first end inserted into the trochanter 68 and a second end clamped between each of the clamping parts 48 and the body 12. A third K-wire 82 passes around the trochanter 68 and around the angled portion 40. This provides an additional securing of the first end 14 of the body to the femur.

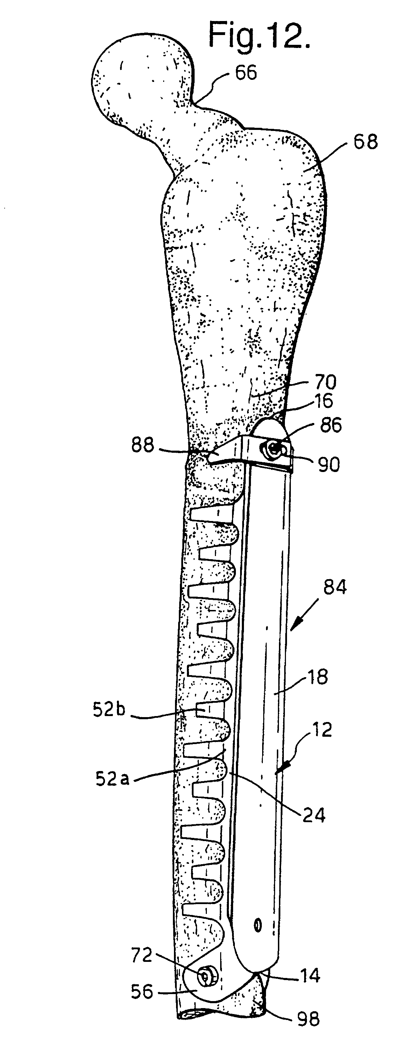

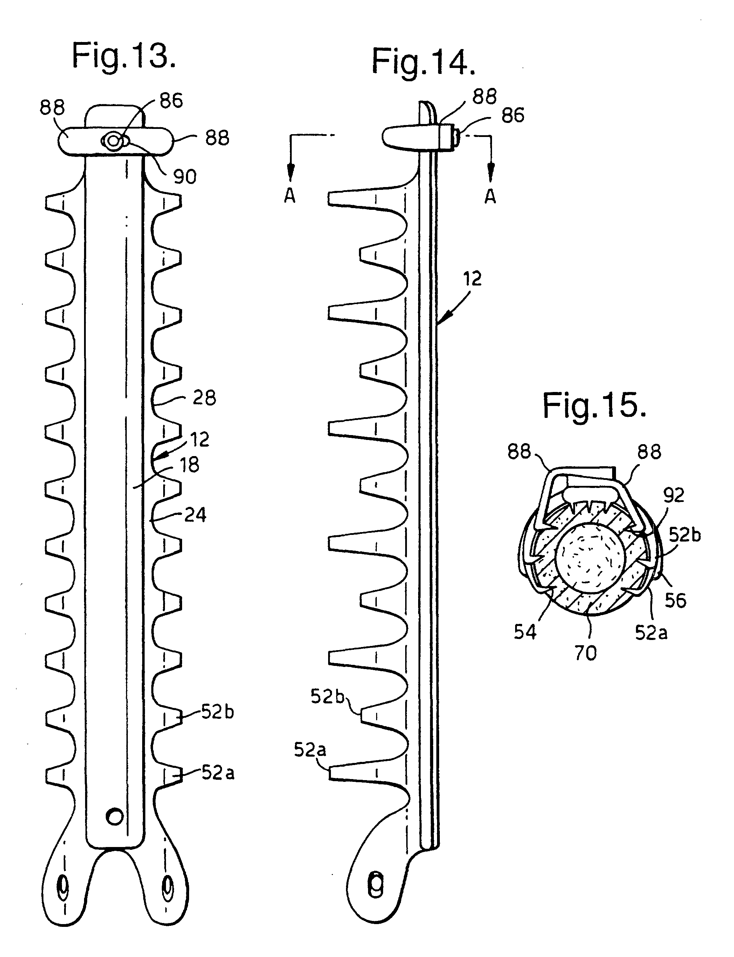

A fourth fracture fixation device 84 is shown in Figures 12 to 15. Parts of the fourth device 84 common to the first device 10 are given the same reference numerals and are not described in detail. The fourth fixation device 84 is suitable for fixing femoral fractures located distal from the prosthesis 66 - for example, in the middle of the femoral shaft 70. In this case, there is less risk of damaging the cement.

The fourth fixation device 84 differs from the first fixation device 10 by the omission of the angled portion 40 and by the omission of the two threaded holes 44, the screws 46 and the clamping parts 48. Instead, the central portion 18 is provided, near the second end 16, with a single threaded hole (not shown). A screw 86 is engaged with the hole and holds first and second generally L-shaped members 88 to the body 12. Each member 88 has a first arm provided with a slot 90 through which the screw 86 passes and a second arm having a spike 92. The spikes 92 penetrate the femoral shaft 70 to lock the second end 16 to the shaft 70. The slots 90 allow the members 88 to be moved relative to the body 12 (when the screw 86 is loosened) so as to accommodate femurs of different widths and so as to allow the members to be positioned with the spikes 92 penetrating the femur. As the slots 90 allow the positions of the members 88 to be adjusted, the members 88 do not need to be flexible. Accordingly, the members are relatively rigid and this allows the members 88 to secure the body 12 to the femur 64 more firmly than the flexible fingers 52a, 52b secure the body 12 to the femur.

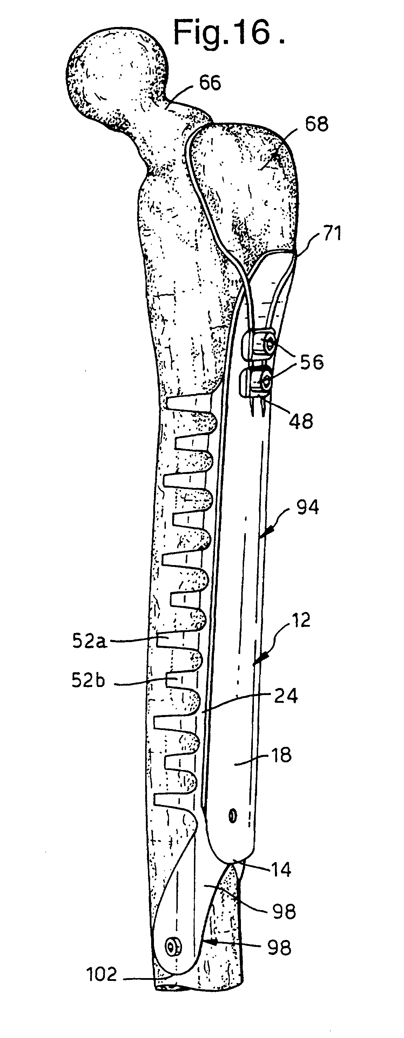

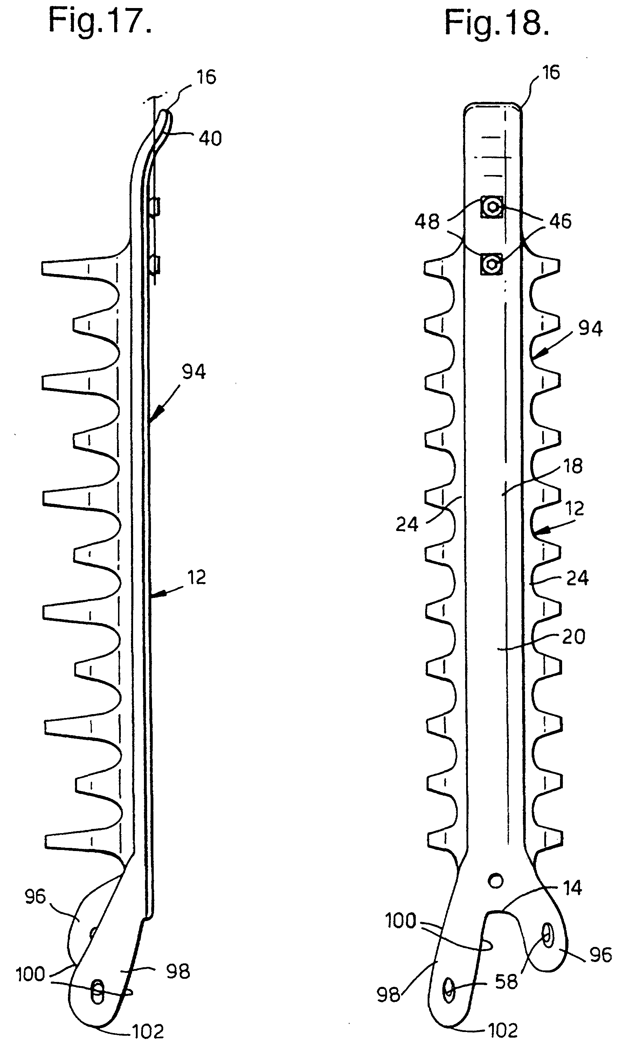

A fifth fixation device 94 is shown in Figures 16 to 19. Parts of the fifth device 94 common to the first device 10 are given the same reference numerals and are not described in detail. The fifth device 94 differs from the first device 10 in that, instead of having two projections 56 of similar shape and size, the device 94 has a first projection 96, similar to the projection 56 of the first device 10, and a second projection 98 that is longer than the first projection 96. The second projection 98 has generally parallel side edges 100 connected by a curved, end edge 102 and extends away from the from end 14 of the body 12 and to the same side of the body 12 as the spikes 30 (not shown). As shown in Figures 17 and 18, the projections 96, 98 are thicker than the fingers 52a, 52b.

As seen in Figure 18, the dynamic compression holes 58 in the two projections 96, 98 are spaced from one another in a direction parallel to the length of the body 12. This reduces or eliminates any possibility that the respective screws that pass through the holes 96, 98 into the bone should collide or otherwise interfere with one another. The provision of the second longer projection 98 allows the holes 96, 98 to be spaced by a greater distance in a direction parallel to the length of the body 12.

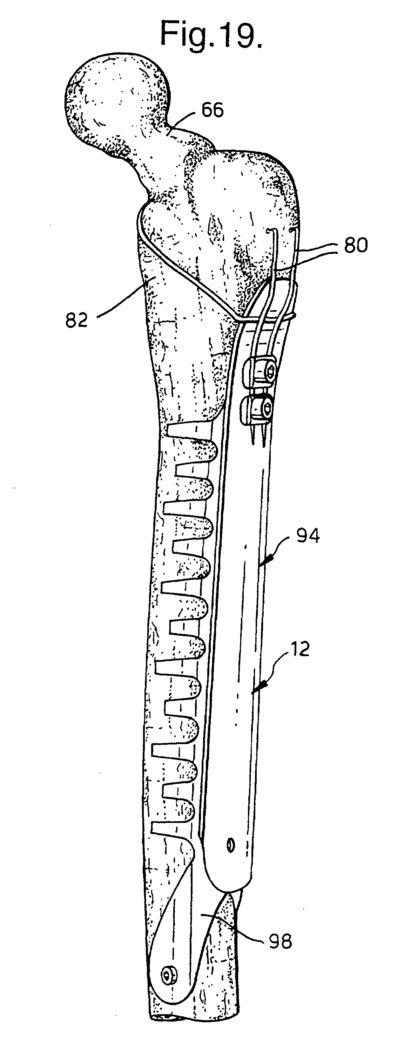

As shown in Figure 19, the fifth fixation device 94 can also be attached at its second end 16 to the femur as described above with reference to Figure 11.

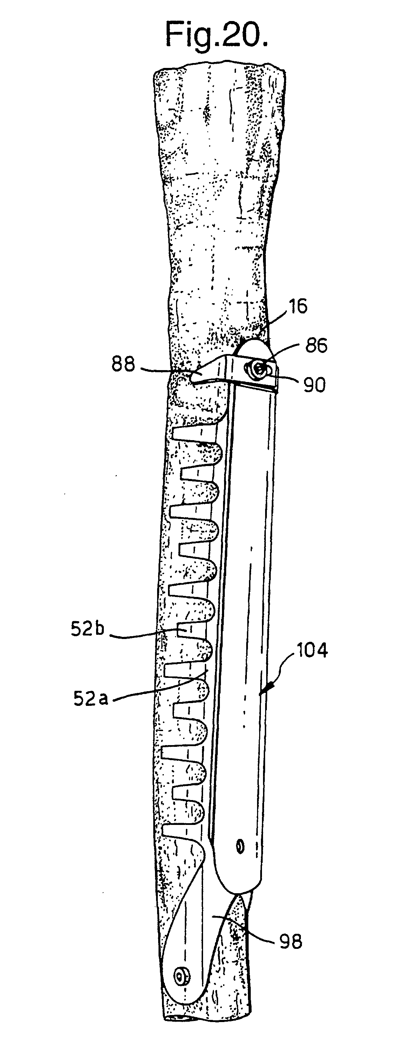

A sixth fixation device 104 is shown in Figure 20. Parts of the sixth device 104 common to the fourth device 84 are given the same reference numerals and are not described in detail. The sixth device 104 is the same as the fourth device 84 with the exception that the sixth device 104 is provided with first and second projections 96, 98, identical to those 96, 98 of the fifth device 94, instead of the two projections 56 of the fourth device 84.

It will be appreciated that the fracture fixation devices may be modified within the scope of the appended claims.

For example, different mechanisms may be used to lock K-wires to the second end 16 of the body 12.

The locking screws 72 may be used with bone inserts where the bone is weakened.

The body 12, the fingers 52a, 52b and the projections 56 do not have to be formed in the shapes described above and shown in the drawings.

## Claims

1. A fracture fixation device (10, 74, 76, 84, 94, 104) for an elongate bone comprising an elongate body (12), first and second rows of fingers (52,52a,52b), each row extending along the body (12) and each finger (52, 52a, 52b) of each row projecting from the body (12), the body (12) being positionable adjacent and along an elongate bone with the fingers (52, 52a, 52b) of the two rows extending around the bone in respective opposite senses to hold the body (12) to the bone, and two projections (56, 96, 98) projecting from the body (12) at or near a first end (14) of the body (12), the projections (56,96,98) extending around the bone in respective opposing senses, **characterized by** each projection (56, 96, 98) having a hole (58) for receiving a respective screw inserted into the bone to lock the projection (56, 96, 98) to the bone.

2. A device (10, 74, 76, 84, 94, 104) according to claim 1, wherein the body (12) has a second end (16) opposed to the first end (14), means (46, 48, 86, 88) being provided for locking the second end (16) to the bone, each hole (58) being shaped so that when said each hole receives a screw inserted into the bone, limited adjustment of the relative positions of said each hole (58) and the screw is possible before the screw is tightened against the corresponding projection (56, 96, 98) to lock said each hole (58) and the screw in an adjusted disposition and to lock the corresponding projection (56,96,98) to the bone, the arrangement allowing a required compressive force to be applied by the body (12) to a fracture in the bone bridged by the body (12).

3. A device (10, 74, 76, 84, 94, 104) according to claim 2, wherein each projection (56,96,98) has a surface portion (62) which, when said each projection (56,96,98) is locked to the bone by a screw, is disposed at an acute angle to the surface of the bone, said locking of said each projection (56,96,98) to the bone comprising seating of the screw against the surface portion (62), the disposition of the surface portion (62) being such as to resist slipping between the screw and the surface portion (62) in a manner that would reduce said compressive force applied to the fracture.

4. A device (10,74,76,84,94,104) according to any one of claims 1 to 3, wherein each projection (56,96,98) extends beyond the first end (14) of the body (12) in a direction parallel to the length of the body (12).

5. A device (10,74,76,84,94,104) according to claim 3, wherein the or each surface portion (62) is part of a surface (60) or a respective surface (60) extending around and facing into the hole (58) or a corresponding one of the holes (58), the portion (62) being located at a side of the hole (58) that is furthermost from the second end (16) in a direction parallel to the length of the body (12).

6. A device (10,74,76,94) according to any one of claims 2, 3 and 5, wherein said second end locking means (46,48) comprises a mechanism for securing a wire.

7. A device (10,74,76,94) according to claim 6, wherein said mechanism (46,48) secures the wire by clamping the wire.

8. A device (10,74,76,94) according to claim 7, wherein said mechanism comprises a screw (46) engaged with the body and holding a clamping part (48), with the wire being clamped between the part (48) and the body (12).

9. A device (10,74,76,94) according to any one of claims 6 to 8, including a wire (71) for passing around the bone.

10. A device (10,74,76,94) according to any one of claims 6 to 8, including a wire (80) for inserting into the bone.

11. A device (84,104) according to any one of claims 2, 3 and 5, wherein said second end locking means comprises a member (88) held by the body (12) and having a sharp formation (92), the member (88) being lockable to the body (12) in a plurality of dispositions so that the member (88) may be locked to the body (12) with a formation (92) penetrating the bone so as to hold the body (12) to the bone.

12. A device (84,104) according to claim 11, wherein the member (88) is held to the body (12) by a screw (86) passing through a slot (90) in the member (88) and engaging the body (12).

13. A device (84,104) according to claim 12, wherein the member (88) has two limbs, the slot (90) being provided on one limb and the sharp formation (92) being provided on the other limb.

14. A device (84,104) according to any one of claims 11 to 13, wherein the member (88) is more rigid than the fingers (52a, 52b).

15. A device (10,74,76,84,94,104) according to any preceding claim, wherein the body (12) has a surface (22) which, when the body (12) is so positioned adjacent and along the bone, faces the bone, said body surface (22) being provided with at least one projection (30) for penetrating the bone.

16. A device (10,74,76,84,94,104) according to claim 15, wherein the body surface (22) is provided with a plurality of projections (30) for penetrating the bone, the projections (30) being spaced from one another along the body (12).

17. A device (10,74,76,84,94,104) according to claim 15 or claim 16, wherein the or each bone penetrating projection (30) includes a formation (34) for limiting penetration of the projection (30) into the bone so as to prevent or limit contact of the body surface (22) with the bone.

18. A device (10,74,76,84,94,104) according to claim 17, wherein the or each bone penetrating projection is a spike (30) having a conical portion (32) for penetrating the bone and a frustro-conical portion (34) for limiting penetration of the spike (30) into the bone, the conical portion (32) extending continuously from the smaller diameter end of the frustro-conical portion (34) and said portions being co-axial, the cone angle of the frustro-conical portion (34) being greater than the cone angle of the conical portion (32).

19. A device (10,76,84,94,104) according to any preceding claim, wherein each finger (52a,52b) of each row has a respective formation (54) for penetrating the bone, each row of fingers having at least one finger (52b) having said formation (54) spaced from the body (12) by a shorter distance along the finger (52b) and at least one finger (52a) having said formation (54) spaced from the body (12) by a longer distance along the finger (52a).

20. A device (10,76,84,94,104) according to claim 19, wherein each row of fingers (52a, 52b) has two or more fingers (52b) having said respective formations (54) spaced from the body (12) by a shorter distance along the fingers (52b) and two or more fingers (52a) having said respective formations (54) spaced from the body (12) by a longer distance along the fingers (52a), said fingers (52b) having the formations (54) spaced from the body (12) by the shorter distance and said fingers (52a) having the formations (54) spaced from the body (12) by the longer distance alternating along the body (12).

21. A device (10, 76, 84, 94, 104) according to claim 20, wherein said fingers (52a) having the formations (54) that are spaced from the body (12) by the longer distance are longer than said fingers (52b) having the formations (54) that are spaced from the body (12) by the shorter distance, each formation (54) being provided at the end of the corresponding finger (52a,52b) remote from the body (12).

22. A device (10,74,76,84,94,104) according to any preceding claim, wherein the two rows are parallel.

23. A device (10,74,76,84,94,104) according to any preceding claim, wherein the body (12) has two parallel, opposed side edges (28), the fingers (52,52a,52b) of one of the rows extending from one of the side edges (28) and the fingers (52,52a,52b) of the other one of the rows extending from the other one of the side edges (28).

24. A device (10, 74, 76, 84, 94, 104) according to claim 23, wherein one of the projections (56) having a hole (58) extends from one of said side edges (28) and the other one of the projections (56) having a hole (58) extends from the other one of said side edges (28).

25. A device (10,74,76,94) according to any one of claims 2 to 4, wherein the body (12) is bent near the second end to form a portion (40) that extends away from the fingers (52,52a,52b).

26. A device (76) according to any preceding claim, wherein the body (12) includes a rib (78) for stiffening the body (12).

## Patentansprüche

1. Bruchfixiervorrichtung (10, 74, 76, 84, 94, 104) für einen länglichen Knochen, der einen länglichen Körper (12), eine erste und eine zweite Reihe von Fingern (52, 52a, 52b), wobei sich jede Reihe entlang des Körpers (12) erstreckt und jeder Finger (52, 52a, 52b) jeder Reihe von dem Körper (12) vorsteht und der Körper (12) an einen länglichen Knochen angrenzend und an ihm entlang positioniert werden kann, wobei sich der Finger (52, 52a, 52b) der zwei Reihen in jeweils entgegengesetzten Richtungen um den Knochen herum erstrecken, um den Körper (12) an dem Knochen zu halten, und zwei Vorsprünge (56, 96, 98) umfasst, die von dem Körper (12) an einem ersten Ende (14) des Körpers (12) oder in dessen Nähe vorstehen, wobei sich die Vorsprünge (56, 96, 98) in jeweils entgegengesetzten Richtungen um den Knochen herum erstrecken, **dadurch gekennzeichnet, dass** jeder Vorsprung (56, 96, 98) ein Loch (58) zum Aufnehmen einer entsprechenden Schraube hat, die in den Knochen eingeführt wird, um den Vorsprung (56, 96, 98) an dem Knochen zu arretieren.

2. Vorrichtung (10, 74, 76, 84, 94, 104) nach Anspruch 1, wobei der Körper (12) ein zweites Ende (16) hat, das dem ersten Ende gegenüberliegt, und Einrichtungen (46, 48, 86, 88) zum Arretieren des zweiten Endes (16) an dem Knochen vorhanden sind, wobei jedes Loch (58) so geformt ist, dass, wenn jedes Loch eine in den Knochen eingeführte Schraube aufnimmt, begrenzte Verstellung der relativen Positionen jedes Lochs (58) und der Schraube möglich ist, bevor die Schraube an dem entsprechenden Vorsprung (56, 96, 98) angezogen wird, um jedes Loch (58) und die Schraube in einer eingestellten Stellung zu arretieren und den entsprechenden Vorsprung (56, 96, 98) an dem Knochen zu arretieren, wobei die Anordnung es ermöglicht, dass eine erforderliche Druckkraft durch den Körper (12) auf einen Bruch in dem Knochen ausgeübt wird, der durch den Körper (12) überbrückt wird.

3. Vorrichtung (10, 74, 76, 84, 94, 104) nach Anspruch 2, wobei jeder Vorsprung (56, 96, 98) einen Oberflächenabschnitt (62) hat, der, wenn jeder Vorsprung (56, 96, 98) mit einer Schraube an dem Knochen arretiert ist, in einem spitzen Winkel zur Oberfläche des Knochens angeordnet ist, wobei das Arretieren jedes Vorsprungs (56, 96, 98) an dem Knochen das Aufsetzen der Schraube auf den Oberflächenabschnitt (62) umfasst und die Stellung des Oberflächenabschnitts (62) so ist, dass Rutschen der Schraube und des Oberflächenabschnitts (62) zueinander, durch das die auf den Bruch ausgeübte Druckkraft verringert würde, Widerstand entgegengesetzt wird.

4. Vorrichtung (10, 74, 76, 84, 94, 104) nach einem der Ansprüche 1 bis 3, wobei sich jeder Vorsprung (56, 96, 98) in einer Richtung parallel zur Länge des Körpers (12) über das erste Ende (14) des Körpers (12) hinaus erstreckt.

5. Vorrichtung (10, 74, 76, 84, 94, 104) nach Anspruch 3, wobei der bzw. jeder Oberflächenabschnitt (62) Teil einer Oberfläche (60) oder einer entsprechenden Oberfläche (60) ist, die sich um das Loch (58) oder ein entsprechendes der Löcher (58) herum erstreckt und ihm zugewandt ist, und sich der Abschnitt (62) an einer Seite des Lochs (58) befindet, die in einer Richtung parallel zur Länge des Körpers (12) am Weitesten von dem zweiten Ende (16) entfernt ist.

6. Vorrichtung (10, 74, 76, 94) nach einem der Ansprüche 2, 3 und 5, wobei die Arretiereinrichtung (46, 48) des zweiten Endes einen Mechanismus zum Befestigen eines Drahtes umfasst.

7. Vorrichtung (10, 74, 76, 94) nach Anspruch 6, wobei der Mechanismus (46, 48) einen Draht durch Klemmen des Drahtes befestigt.

8. Vorrichtung (10, 74, 76, 94) nach Anspruch 7, wobei der Mechanismus eine Schraube (46) umfasst, die mit dem Körper in Eingriff ist und ein Klemmteil (48) hält, und der Draht zwischen dem Teil (48) und dem Körper eingeklemmt wird.

9. Vorrichtung (10, 74, 76, 94) nach einem der Ansprüche 6 bis 8, die einen Draht (41) zum Herumführen um den Knochen enthält.

10. Vorrichtung (10, 74, 76, 94) nach einem der Ansprüche 6 bis 8, die einen Draht (80) zum Einführen in den Knochen enthält.

11. Vorrichtung (84, 104) nach einem der Ansprüche 2, 3 und 5, wobei die Arretiereinrichtung des zweiten Endes ein Element (88) umfasst, das von dem Körper (12) gehalten wird und eine scharfe Struktur (92) hat, wobei das Element (88) an dem Körper (12) in einer Vielzahl von Stellungen arretiert werden kann, so dass das Element (88) so an dem Körper (12) arretiert werden kann, dass eine Struktur (92) in den Knochen eindringt, um den Körper (12) an dem Knochen zu halten.

12. Vorrichtung (84, 104) nach Anspruch 11, wobei das Element (88) an dem Körper (12) von einer Schraube (86) gehalten wird, die durch einen Schlitz (90) in dem Element (88) hindurchtritt und mit dem Körper (12) in Eingriff kommt.

13. Vorrichtung (84, 104) nach Anspruch 12, wobei das Element (88) zwei Schenkel hat und der Schlitz (90) an einem Schenkel vorhanden ist und die scharfe Struktur (92) an dem anderen Schenkel vorhanden ist.

14. Vorrichtung (84, 104) nach einem der Ansprüche 11 bis 13, wobei das Element (88) starrer ist als die Finger (52a, 52b).

15. Vorrichtung (10, 74, 76, 84, 94, 104) nach einem der vorangehenden Ansprüche, wobei der Körper (12) eine Oberfläche (22) hat, die, wenn der Körper (12) an dem Knochen angrenzend und an ihm entlang positioniert ist, dem Knochen zugewandt ist, wobei die Körper-Oberfläche (22) mit wenigstens einem Vorsprung (30) zum Eindringen in den Knochen versehen ist.

16. Vorrichtung (10, 74, 76, 84, 94, 104) nach Anspruch 5, wobei die Körper-Oberfläche (22) mit einer Vielzahl von Vorsprüngen (33) zum Eindringen in den Knochen versehen ist und die Vorsprünge (30) entlang des Körpers (12) voneinander beabstandet sind.

17. Vorrichtung (10, 74, 76, 84, 94, 104) nach Anspruch 15 oder Anspruch 16, wobei der bzw. jeder Knochen-Eindringvorsprung (30) eine Struktur (34) zum Begrenzen des Eindringens des Vorsprungs (30) in den Knochen enthält, um Kontakt der Körperoberfläche (22) mit dem Knochen zu verhindern oder zu begrenzen.

18. Vorrichtung (10, 74, 76, 84, 94, 104) nach Anspruch 17, wobei der bzw. jeder Knochen-Eindringvorsprung ein Dorn (30) mit einem kegelförmigen Abschnitt (32) zum Eindringen in den Knochen und einen kegelstumpfförmigen Abschnitt (34) zum Begrenzen des Eindringens des Dorns (30) in den Knochen ist, der kegelförmige Abschnitt (32) sich durchgehend von dem Ende des kegelstumpfförmigen Abschnitts (34) mit kleinerem Durchmesser erstreckt und die Abschnitte koaxial sind, wobei der Kegelwinkel des kegelstumpfförmigen Abschnitts (34) größer ist als der Kegelwinkel des kegelförmigen Abschnitts (32).

19. Vorrichtung (10, 76, 84, 94, 104) nach einem der vorangehenden Ansprüche, wobei jeder Finger (52a, 52b) jeder Reihe eine entsprechende Struktur (54) zum Eindringen in den Knochen hat und jede Reihe von Fingern wenigstens einen Finger (52b), bei dem die Struktur (54) um eine kürzere Strecke entlang des Fingers (52b) von dem Körper (12) beabstandet ist, und wenigstens einen Finger (52a) hat, bei dem die Struktur (54) um eine längere Strecke entlang des Fingers (52a) von dem Körper (12) beabstandet ist.

20. Vorrichtung (10, 76, 84, 94, 104) nach Anspruch 19, wobei jede Reihe von Fingern (52a, 52b) zwei oder mehr Finger (52b), bei denen die entsprechenden Strukturen (54) um eine kürzere Strecke entlang der Finger (52b) von dem Körper (12) beabstandet sind, und zwei oder mehr Finger (52a) hat, bei denen die entsprechenden Strukturen (54) um eine längere Strecke entlang der Finger (52a) von dem Körper (12) beabstandet sind, wobei sich die Finger (52b), bei denen die Strukturen (54) um die kürzere Strecke von dem Körper (12) beabstandet sind, und die Finger (52a), bei denen die Strukturen (54) um die längere Strecke von dem Körper (12) beabstandet sind, entlang des Körpers (12) abwechseln.

21. Vorrichtung (10, 76, 84, 94, 104) nach Anspruch 20, wobei die Finger (52a) mit den Strukturen (54), die um die längere Strecke von dem Körper (12) beabstandet sind, länger sind als die Finger (52a) mit den Strukturen (54), die um die kürzere Strecke von dem Körper (12) beabstandet sind, und jede Struktur (54) an dem Ende des entsprechenden Fingers (52a, 52b) vorhanden ist, das von dem Körper (12) entfernt ist.

22. Vorrichtung (10, 74, 76, 84, 94, 104) nach einem der vorangehenden Ansprüche, wobei die zwei Reihen parallel sind.

23. Vorrichtung (10, 74, 76, 84, 94, 104) nach einem der vorangehenden Ansprüche, wobei der Körper (12) zwei parallele, einander gegenüberliegende Seitenkanten (28) hat und sich die Finger (52, 52a, 52b) einer der Reihen von einer der Seitenkanten (28) aus erstrecken und sich die Finger (52, 52a, 52b) der anderen der Reihen von der anderen der Seitenkanten (28) aus erstrecken.

24. Vorrichtung (10, 74, 76, 84, 94, 104) nach Anspruch 23, wobei sich einer der Vorsprünge (56), der ein Loch hat (58), von einer der Seitenkanten (28) aus erstreckt und sich der andere der Vorsprünge (56), der ein Loch (58) hat, von der anderen Seitenkante (28) aus erstreckt.

25. Vorrichtung (10, 74, 76, 94) nach einem der Ansprüche 2 bis 4, wobei der Körper (12) in der Nähe des zweiten Endes gebogen ist, um einen Abschnitt (40) auszubilden, der sich von den Fingern (52, 52a, 52b) weg erstreckt.

26. Vorrichtung (76) nach einem der vorangehenden Ansprüche, wobei der Körper (12) eine Rippe (78) zum Versteifen des Körpers (12) enthält.

## Revendications

1. Dispositif de fixation de fracture (10, 74, 76, 84, 94, 104) pour un os allongé, comprenant un corps allongé (12), une première et une seconde rangée de doigts (52, 52a, 52b), chaque rangée s'étendant le long du corps (12) et chaque doigt (52, 52a, 52b) de chaque rangée se projetant depuis le corps (12), le corps (12) pouvant être positionné adjacent et le long d'un os allongé avec les doigts (52, 52a, 52b) des deux rangées s'étendant autour de l'os dans des sens respectifs opposés pour tenir le corps (12) sur l'os, et deux projections (56, 96, 98) qui se projettent depuis le corps (12) à une première extrémité (14) du corps (12) ou au voisinage de cette extrémité, les projections (56, 96, 98) s'étendant autour de l'os dans des sens respectifs opposés, **caractérisé en ce que** chaque projection (56, 96, 98) comporte un trou (58) pour recevoir une vis respective introduite dans l'os pour bloquer la projection (56, 96, 98) sur l'os.

2. Dispositif (10, 74, 76, 84, 94, 104) selon la revendication 1, dans lequel le corps (12) possède une seconde extrémité (16) opposée à la première extrémité (14), des moyens (46, 48, 86, 88) étant prévus pour bloquer la seconde extrémité (16) sur l'os, chaque trou (58) étant ainsi conformé que lorsque chacun desdits trous reçoit une vis introduite dans l'os, un ajustement limité des positions relatives dudit trou (58) et de ladite vis est possible avant de serrer la vis contre la projection correspondante (56, 96, 98) pour bloquer ledit trou (58) et ladite vis dans une disposition ajustée et pour bloquer la projection correspondante (56, 96, 98) sur l'os, l'agencement permettant d'appliquer une force de compression requise par le corps (12) à une fracture dans l'os ponté par le corps (12).

3. Dispositif (10, 74, 76, 84, 94, 104) selon la revendication 2, dans lequel chaque projection (56, 96, 98) possède une portion de surface (62) qui, quand chacune desdites projections (56, 96, 98) est bloquée sur l'os par une vis, est disposée sous un angle aigu par rapport à la surface de l'os, ledit blocage de chacune desdites projections (56, 96, 98) sur l'os comprenant d'appliquer la vis contre la portion de surface (62), la disposition de la portion de surface (62) étant propre à résister à un glissement entre la vis et la portion de surface (62) d'une manière qui pourrait réduire ladite force de compression appliquée à la fracture.

4. Dispositif (10, 74, 76, 84, 94, 104) selon l'une quelconque des revendications 1 à 3, dans lequel chaque projection (56, 96, 98) s'étend au-delà de la première extrémité (14) du corps (12) dans une direction parallèle à la longueur du corps (12).

5. Dispositif (10, 74, 76, 84, 94, 104) selon la revendication 3, dans lequel la portion de surface (62) ou chaque portion de surface fait partie d'une surface (60) ou d'une surface respective (60) s'étendant autour de et faisant face vers l'intérieur du trou (58) ou d'un trou correspondant parmi les trous (58), la portion (62) étant située sur un côté du trou (58) qui est le plus éloigné de la seconde extrémité (16) dans une direction parallèle à la longueur du corps (12).

6. Dispositif (10, 74, 76, 94) selon l'une quelconque des revendications 2, 3 et 5, dans lequel lesdits moyens de blocage (46, 48) pour la seconde extrémité comprennent un mécanisme pour fixer un fil.

7. Dispositif (10, 74, 76, 94) selon la revendication 6, dans lequel ledit mécanisme (46, 48) fixe le fil en pinçant le fil.

8. Dispositif (10, 74, 76, 94) selon la revendication 7, dans lequel ledit mécanisme comprend une vis (46) engagée avec le corps et maintenant une partie de pincement (48), le fil étant pincé entre la partie (48) et le corps (12).

9. Dispositif (10, 74, 76, 94) selon l'une quelconque des revendications 6 à 8, comprenant un fil (71) destiné à passer autour de l'os.

10. Dispositif (10, 74, 76, 94) selon l'une quelconque des revendications 6 à 8, comprenant un fil (80) destiné à être introduit dans l'os.

11. Dispositif (94, 104) selon l'une quelconque des revendications 2, 3 et 5, dans lequel lesdits moyens de blocage à la seconde extrémité comprennent un élément (88) maintenu par le corps (12) et possédant une formation acérée (92) ledit élément (88) pouvant être bloqué sur le corps (12) dans une pluralité de dispositions de telle façon que le corps (88) peut être bloqué sur le corps (12) avec la formation (92) en pénétration dans l'os de manière à tenir le corps (12) sur l'os.

12. Dispositif (84, 104) selon la revendication 11, dans lequel l'élément (88) est tenu sur le corps (12) par une vis (86) qui traverse une fente (90) dans l'élément (88) et qui engage le corps (12).

13. Dispositif (84, 104) selon la revendication 12, dans lequel l'élément (82) possède deux membres, la fente (90) étant prévue sur l'un des membres, et la formation acérée (92) étant prévue sur l'autre membre.

14. Dispositif (84, 104) selon l'une quelconque des revendications 11 à 13, dans lequel l'élément (88) est plus rigide que les doigts (52a, 52b).

15. Dispositif (10, 74, 76, 84, 94, 104) selon l'une quelconque des revendications précédentes, dans lequel le corps (12) possède une surface (22) qui, quand le corps (12) est positionné adjacent et le long de l'os, fait face vers l'os, ladite surface (22) du corps étant pourvue d'au moins une projection (30) pour pénétrer l'os.

16. Dispositif (10, 74, 76, 84, 94, 104) selon la revendication 15, dans lequel la surface (22) du corps est pourvue d'une pluralité de projections (30) pour pénétrer l'os, les projections (30) étant espacées les unes des autres le long du corps (12).

17. Dispositif (10, 74, 76, 84, 94, 104) selon l'une ou l'autre des revendications 15 et 16, dans lequel la projection de pénétration d'os (30) ou chaque projection inclut une formation (34) pour limiter la pénétration de la projection (30) dans l'os de manière à empêcher ou limiter le contact de la surface (22) du corps avec l'os.

18. Dispositif (10, 74, 76, 84, 94, 104) selon la revendication 17, dans lequel la projection de pénétration d'os ou chaque projection est une pointe (30) ayant une portion conique (32) pour pénétrer l'os et une portion tronconique (34) pour limiter la pénétration de la pointe (30) dans l'os, la portion conique (32) s'étendant en continu depuis l'extrémité de petit diamètre de la portion de tronconique (34), et lesdites portions étant coaxiales, l'angle du cône de la portion tronconique (34) étant supérieur à l'angle du cône de la portion conique (32).

19. Dispositif (10, 76, 84, 94, 104) selon l'une quelconque des revendications précédentes, dans lequel chaque doigt (52a, 52b) de chaque rangée possède une formation respective (54) pour pénétrer l'os, chaque rangée de doigts ayant au moins un doigt (52b) ayant ladite formation (54) espacée depuis ledit corps (12) d'une distance plus courte le long du doigt (52b), et au moins un doigt (52a) ayant ladite formation (54) espacée depuis ledit corps (12) d'une distance plus longue le long du doigt (52a).

20. Dispositif (10, 76, 84, 94, 104) selon la revendication 19, dans lequel chaque rangée de doigts (52a, 52b) possède deux ou plusieurs doigts (52b) ayant lesdites formations respectives (54) espacées depuis ledit corps (12) d'une distance plus courte le long des doigts (52b) et deux ou plusieurs doigts (52a) ayant lesdites formations respectives (54) espacées depuis ledit corps (12) d'une distance plus longue le long des doigts (52a), lesdits doigts (52b) qui comportent les formations (54) espacées depuis le corps (12) sur la plus courte distance et lesdits doigts (52a) qui comportent les formations (54) espacées depuis le corps (12) sur la plus longue distance alternant le long du corps (12).

21. Dispositif (10, 76, 84, 94, 104) selon la revendication 20, dans lequel lesdits doigts (52a) qui comportent les formations (54) espacées depuis le corps (12) sur la plus longue distance sont plus longs que lesdits doigts (52b) qui comportent les formations (54) espacées depuis le corps (12) sur la plus courte distance, chaque formation (54) étant prévue à l'extrémité du doigt correspondant (52a, 52b) éloignée depuis le corps (12).

22. Dispositif (10, 74, 76, 84, 94, 104) selon l'une quelconque des revendications précédentes, dans lequel les deux rangées sont parallèles.

23. Dispositif (10, 74, 76, 84, 94, 104) selon l'une quelconque des revendications précédentes, dans lequel le corps (12) possède deux bords latéraux parallèles opposés (28), les doigts (52, 52a, 52b) de l'une des rangées s'étendant depuis l'un des bords latéraux (28) et les doigts (52, 52a, 52b) de l'autre rangée s'étendant depuis l'autre des bords latéraux (28).

24. Dispositif (10, 74, 76, 84, 94, 104) selon la revendication 23, dans lequel l'une des projections (56) possédant un trou (58) s'étend depuis l'un desdits bords latéraux (28), et l'autre projection (56) possédant un trou (58) s'étend depuis l'autre desdits bords latéraux (28).

25. Dispositif (10, 74, 76, 94) selon l'une quelconque des revendications 21 4, dans lequel le corps (12) est cintré au voisinage de la seconde extrémité pour former une portion (40) qui s'étend en éloignement des doigts (52, 52a, 52b).

26. Dispositif (76) selon l'une quelconque des revendications précédentes, dans lequel le corps (12) inclut une nervure (78) pour rigidifier le corps (12).
